(19) Europäisches Patentamt
European Patent Office
Office européen des brevets

(11) EP 1 413 589 A1

(12) **EUROPEAN PATENT APPLICATION**
published in accordance with Art. 158(3) EPC

(43) Date of publication:
**28.04.2004 Bulletin 2004/18**

(21) Application number: **02745977.5**

(22) Date of filing: **11.07.2002**

(51) Int Cl.[7]: **C08F 290/06**, A61K 7/16, A61K 31/80, A61P 1/02, A61P 31/04, A61K 47/32

(86) International application number:
**PCT/JP2002/007066**

(87) International publication number:
**WO 2003/006519 (23.01.2003 Gazette 2003/04)**

(84) Designated Contracting States:
**AT BE BG CH CY CZ DE DK EE ES FI FR GB GR IE IT LI LU MC NL PT SE SK TR**

(30) Priority: **13.07.2001 JP 2001213783**
**01.08.2001 JP 2001233390**

(71) Applicant: **Kao Corporation**
**Tokyo 103-8210 (JP)**

(72) Inventors:
- **KOUDATE, Takashi, Kao Corporation**
  **Wakayama-shi, Wakayama 640-8580 (JP)**
- **SHIMOTOYODOME, Akira, Kao Corporation**
  **Haga-gun, Tochigi 321-3426 (JP)**

(74) Representative: **HOFFMANN - EITLE**
**Patent- und Rechtsanwälte**
**Arabellastrasse 4**
**81925 München (DE)**

(54) **POLYMERS FOR ORAL USE AND COMPOSITIONS FOR ORAL USE**

(57) The present invention provides an oral-use polymer that is provided with a polymer chain (hereinafter, referred to as polymer chain 1) containing a repetitive structure derived from a monomer having a residue obtained by removing one hydrogen atom from one kind or more of phosphorus-containing acids selected from the group consisting of phosphoric acid, polyphosphoric acid, phosphonic acid, polyphosphonic acid, phosphinic acid, partial esters thereof and salts thereof, and

a hydrophilic polymer chain (hereinafter, referred to as polymer chain 2) , as well as a composition of such an oral-use polymer. These make it possible to inhibit adsorption of bacterial onto the tooth surface and also to suppress formation of bacterial plaque.



Printed by Jouve, 75001 PARIS (FR)

## Description

Field of the Invention

The present invention relates to a polymer that is effectively used as an oral care agent, and useful for an oral-care application that suppresses adhesion of bacteria to the surface of a tooth, and also relates to an oral-use composition containing such a polymer.

Background Art

It has been conventionally known that bacterial plaque causes periodontal diseases and decayed teeth, and when the bacterial plaque is left untreated, it turns into dental calcium (crystalline calcium) that is difficult to remove. The formation of dental calcium starts when protein in saliva is adsorbed onto the surface of a tooth to form a coat referred to as pellicle and bacteria are adsorbed onto this pellicle. With respect to the suppresser agent against the bacterial plaque formation, conventionally-used germicides and antibacterial agents have been reported as effective agents for reducing the number of intraoral bacteria (J. Periodontol 1991 62 (11) : 649 to 651) and the like; however, it is difficult to maintain its effective concentration in the oral cavity due to a washing function of saliva, and the effects are insufficient (Oral Surg Oral Med. Oral Pathol 1990 Apr. ; 69(4) : 444 to 449). Moreover, in the case where dental calcium has already been present, these agents might reduce the metabolic activity of bacteria in the dental calcium, thereby accelerating mineral deposition, that is, calcification.

With respect to the dental calcium suppresser agent, the application of phosphated starch (JP-A No. 4-217613), the combined application of alginic acid and divalent metal (JP-A No. 9-175968) and the like have been proposed. These agents are only used for nosotropic purposes to prevent calcium phosphate and the like in bacterial plaque from being crystallized to turn into dental calcium.

JP-A No. 9-175965 has disclosed that a composition, which is made from a polymer selected from copolymers between a homopolymer of dimethyl diallyl ammonium chloride and a polymerizable monomer having dimethyl diallyl ammonium chloride and at least one kind of ethylenic unsaturated hydrocarbon bond, and a betaine-type surfactant, is effective in suppressing adhesion of bacteria onto the surface of a tooth. Moreover, JP-A No. 57-135817 has disclosed that a water-soluble copolymer constituted by units having a molecular structure of units derived from vinyl phosphonic acid and units derived from vinyl phosphonyl fluoride makes it possible to prevent bacteria from being adsorbed onto saliva treatment coat hydroxy apatite beads. However, these have not provided sufficient effects.

DISCLOSURE OF THE INVENTION

The present invention relates to a bacteria adsorption inhibitor that can inhibit adsorption of bacteria onto the surface of a tooth effectively, a bacterial-plaque-formation suppresser agent that suppresses formation of pellicle, that is, adsorption of saliva protein onto the surface of tooth enamel, and formation of bacterial plaque, and an oral-use composition which prevents occurrence of oral diseases caused by dental plaque.

The present inventors have found that a polymer, which has a polymer chain having a functional group that exerts an adsorbing property to teeth and a hydrophilic polymer chain, exerts a high bacteria adsorption inhibiting effect and an oral-plaque-formation suppressing effect.

The present invention provides an oral-use polymer which has a polymer chain (hereinafter, referred to as polymer chain 1) containing a repetitive structure derived from a monomer having a residue (hereinafter, referred to as phosphorous-based acid residue) obtained by removing one hydrogen atom from one kind or more of phosphorus-containing acids selected from the group consisting of phosphoric acid, polyphosphoric acid, phosphonic acid, polyphosphonic acid, phosphinic acid, partial esters thereof and salts thereof, and a hydrophilic polymer chain (hereinafter, referred to as polymer chain 2), in particular, an oral-use polymer represented by the following formula (III), and also provides a bacteria-adsorption inhibitor made from the above-mentioned oral-use polymer, a bacterial-plaque-formation inhibitor made from the above-mentioned oral-use polymer and an oral-use composition containing such an oral-use polymer.

The present invention also provides an application of the above-mentioned polymer as a bacteria-adsorption inhibitor or a bacterial-plaque-formation inhibitor, a method for applying the above-mentioned polymer to the oral cavity so as to inhibit adsorption of bacteria or suppress formation of bacterial plaque, and an oral-use composition containing the above-mentioned polymer and medium.

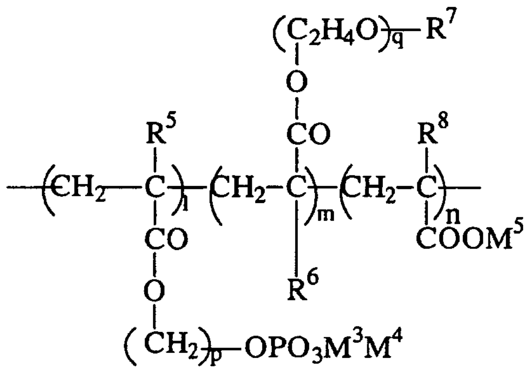

(III)

(wherein, l, m and n are set in the range of l : m : n = 1 to 95 : 5 to 80 : 0 to 94, and 0. 25 ≤ (l + n) / m ≤ 19, in mole % (l + m + n = 100) ; p represents a number of 1 to 22 and q represents a number of 1 to 200 in the number-average value; $R^5$, $R^6$, $R^7$ and $R^8$ independently each represent a monovalent hydrocarbon group that may be substituted by a hydrogen atom or a fluorine atom; $M^3$, $M^4$ and $M^5$ independently represent a hydrogen atom, metal, ammonium, alkyl or alkenyl ammonium having 1 to 22 carbon atoms in total, alkyl or alkenyl substituted pyridinium having 1 to 22 carbon atoms, alkanol ammonium having 1 to 22 carbon atoms in total, or basic amino acid residue)

[Oral-use polymer]

The oral-use polymer of the present invention is constituted by not less than two polymer chains having polymer chain 1 and polymer chain 2.

With respect to the polymer constituted by not less than two polymer chains, examples thereof include a star polymer, a block polymer and a graft polymer; preferably, a block or graft polymer constituted by polymer chain 1 and polymer chain 2, more preferably, a graft polymer, most preferably, a graft polymer having polymer chain 1 as a main chain and polymer chain 2 as a side chain.

The molecular weight ratio (polymer chain 1/polymer chain 2) of polymer chain 1 and polymer chain 2 is preferably set in the range of 10/1 to 1/10, more preferably, 5/1 to 1/5. The ratio in this range provides appropriate adsorbing property and hydrophilic property to a tooth with a preferably high bacteria adsorption inhibiting effect.

The molecular weight of the oral-use polymer of the present invention is preferably set to 5,000 to 1000, 000, more preferably, 6,000 to 1000,000, most preferably, 10,000 to 200,000, in the weight-average molecular weight measured by a measuring method, which will be described later. In this range, the viscosity in the aqueous solution is appropriate, and provides easiness in handling, with a high bacteria-adsorption inhibiting effect.

With respect to the oral-use polymer of the present invention, a polymer having a repetitive structure represented by formula (I) and a repetitive structure represented by formula (II) is particularly preferable.

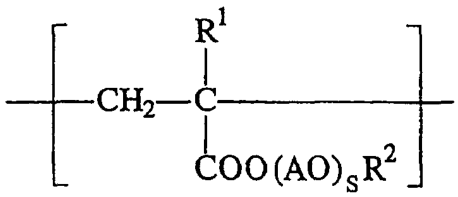

(I)

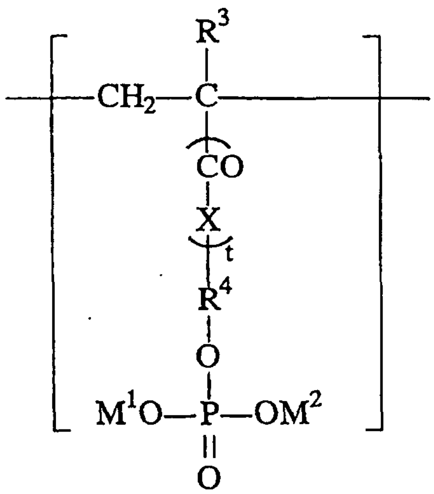

(II)

[In the formulas, $R^1$ and $R^3$, which may be the same or different, represent a monovalent hydrocarbon group that may be substituted by a hydrogen atom or a fluorine atom, $R^2$ represents a monovalent hydrocarbon group that may have a hydrogen atom or a substituent, and $R^4$ represents a divalent hydrocarbon group. AO represents an oxyalkylene group having 2 to 3 carbon atoms, s represents a number of 1 to 200 in the number average value, and n-number of AOs may be the same or different. X represents -O- or -NH-, and t represents 0 or 1. $M^1$ and $M^2$, which may be the same or different, represent a hydrogen atom, metal, ammonium, alkyl or alkenyl ammonium having 1 to 22 carbon atoms in total, alkyl or alkenyl substituted pyridinium having 1 to 22 carbon atoms, alkanol ammonium having 1 to 22 carbon atoms in total, or basic amino acid.]

In formula (I), preferably, $R^1$ is a hydrogen atom or a methyl group, more preferably, a methyl group. Preferably, $R^2$ is a hydrogen atom or an alkyl group or an alkenyl group having a straight chain or a branched chain, which may have a substituent, and has 1 to 22 carbon atoms, and in the case where the alkyl group or the alkenyl group has a substituent, this preferably has a hydrophilic property, with the substituent being prepared as a hydroxy group, a carboxy group, an amino group, an ammonium group, an alkoxy group (having 1 to 4 carbon atoms), an acyl group (having 1 to 8 carbon atoms), a halogen atom (a fluorine atom, a chlorine atom or the like) and the like. Preferably, $R^2$ is a hydrogen atom or an alkyl group having 1 to 8 carbon atoms, and examples thereof include a methyl group, an ethyl group, an i-propyl group, an n-propyl group, a butyl group, a hexyl group and an octyl group. More preferable example is a hydrogen atom or a methyl group.

Here, $(AO)_s$ may be a polyoxy ethylene chain, a polyoxy propylene chain or a mixed chain of an oxyethylene group and an oxypropylene group, and the bonding may be either a random, block or alternate bond. Preferably, this is prepared as a polyoxy ethylene chain. In this case, s represents a number of 1 to 200 in the number average, preferably, 10 to 200, more preferably, 20 to 200, most preferably, 40 to 150.

In formula (II), preferably, $R^3$ is a hydrogen atom or a methyl group, more preferably, a methyl group. $R^4$ is preferably a straight chain or branched chain alkylene group having 1 to 22 carbon atoms, which may have an arylene group such as a phenylene group. More preferably, this is prepared as a straight-chain alkylene group having 2 to 14 carbon atoms, most preferably, a straight-chain alkylene group having 8 to 12 carbon atoms. Preferably, X is -O-. Preferably, t is set to 1, since this portion forms an adsorption site to the tooth. When prepared as alkanol ammonium having 1 to 22 carbon atoms in total, $M^1$ and $M^2$ are preferably monoethanol ammonium, diethanol ammonium and triethanol ammonium, and when prepared as a basic amino acid residue, these are preferably arginine, lysine, histidine, ornithine and the like. More preferably, these are a hydrogen atom or alkali metal such as sodium and potassium, most preferably, a hydrogen atom.

The rate of the repetition structure represented by formula (I) and the repetition structure represented by formula (II) is preferably set to (II)/(I) (weight ratio) = 10/90 to 90/10, more preferably, 10/90 to 50/50, most preferably, 20/80 to 40/60.

[Polymer chain 1]

With respect to polymer chain 1 of the present invention, any polymer chain may be used as long as it contains a repetitive structure derived from a monomer having a phosphorous-based acid residue. With respect to the phosphorous-based acid residue, as described in WO98/26749, examples thereof include a phosphate group or a diphosphate group having at least one hydroxyl group, such as a phosphate residue (1), a phosphoric acid monoester residue (2), a diphosphate residue (3), a diphosphoric acid monoester residue (4) and a diphosphoric acid diester residue (5), or salts thereof; a phosphonate group or diphosphonate group having at least one hydroxyl group, such as a phosphonic acid residue (6), a phosphonic acid monoester residue (7), a diphosphonic acid residue (8) and a diphosphonic acid monoester residue (9), or salts thereof; and a phosphinic acid residue (phosphinate group) (10) or salts thereof. Among the above-mentioned groups (1) to (10), from the viewpoints of availability and safety, the residues (1) to (5) and salts thereof are more preferably used, and in particular, the phosphate residue (1) or salts thereof is most preferably used. One or two or more hydroxyl groups in the phosphorous-based acid residue may be a halide substituted by a chlorine atom, a bromine atom, a fluorine atom or the like.

With respect to the monomer having the phosphorous-based residue, a vinyl monomer having a structure represented by formula (IV) is preferably used.

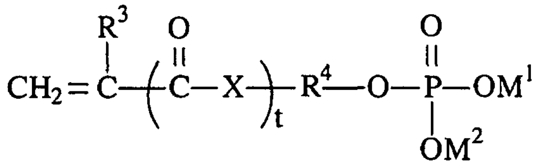

(IV)

[wherein, $R^3$, $R^4$, X, $M^1$, $M^2$ and t are used in the same definitions as the above-mentioned formulas.]

Specific examples thereof include methacryloyloxy ethyl phosphate (Phosmer M, made by Unichemical Co., Ltd.), methacryloyloxy decyl phosphate and the like.

Polymer chain 1 may be a single polymer of a monomer represented by formula (IV), or a copolymer with another polymerizable monomer. With respect to said another monomer, examples thereof include (A) a monomer having an anionic group other than the monomer having a phosphorous-based acid residue, (B) a monomer having a nonionic group and (C) a monomer having a cationic group.

With respect to (A) the monomer having an anionic group, examples thereof include: a carboxylic acid-based monomer having a polymerizable unsaturated group and/or salts thereof, such as (meth)acrylic acid ("(meth)acrylic" refers to methacrylic acid, acrylic acid or a mixture thereof), maleic acid (including maleic anhydride, maleic acid monoester, and maleic acid monoamide or a mixture thereof), itaconic acid (including itaconic anhydride, itaconic acid monoester and itaconic acid monoamide or a mixture thereof) and crotonic acid; and a sulfonic acid-based monomer having a polymerizable unsaturated group and/or salts thereof, such as styrene sulfonic acid and 2-(meth)acrylamide-2-alkyl (having 1 to 4 carbon atoms) propane sulfonic acid (more specifically, 2-acrylamide-2-methylpropane sulfonic acid).

The anionic group may be neutralized to a desired degree of neutralization by a basic substance. In this case, all the anionic groups or one portion of the anionic groups in the polymer generate salts.

Here, with respect to cationic ions in the salts, examples thereof include ammonium ions, trialkyl ammonium ions having 3 to 54 carbon atoms in total (for example, trimethyl ammonium ions, triethyl ammonium ions) , hydroxyalkyl ammonium ions having 2 to 4 carbon atoms, dihydroxyalkyl ammonium ions having 4 to 8 carbon atoms in total, trihydroxyalkyl ammonium ions having 6 to 12 carbon atoms in total, alkali metal ions and alkali earth metal ions. With respect to the neutralization, a monomer may be neutralized or a polymer formed by the monomer may be neutralized.

Among these, preferably, (meth)acrylic acid, crotonic acid, itaconic acid, maleic acid, styrene sulfonic acid, 2-acrylamide-2-methylpropane sulfonic acid and/or the salts thereof are used; more preferably, (meth)acrylic acid, crotonic acid, itaconic acid, maleic acid and/or the salts thereof are used; and most preferably, (meth)acrylic acid and/or the salt thereof are used.

With respect to (B) the monomer having a nonionic group, examples thereof include: vinyl alcohol; (meth)acrylic acid esters or (meth)acryl amides having hydroxy alkyl groups (having 1 to 22 carbon atoms), such as N-hydroxypropyl (meth)acrylate, N-hydroxyethyl (meth)acrylate and N-hydroxypropyl, (meth)acryl amide; (meth)acrylic acid esters of polyether, such as polyethylene glycol (meth)acrylate (degree of polymerization 1 to 30 in ethylene glycol); (meth)acryl amide; alkyl (meth)acryl amides (having 1 to 22 carbon atoms), such as N-methyl(meth)acryl amide, N-n-propyl(meth)acryl amide, N-isopropyl (meth)acryl amide, N-t-butyl (meth)acryl amide and N-isobutyl (meth)acryl amide; dialkyl (meth)acryl amides (having 2 to 44 carbon atoms in total), such as N, N-dimethyl (meth) acryl amide and N, N-diethyl (meth)acryl amide; diacetone (meth)acryl amide; N-vinyl cyclic amides such as N-vinyl pyrrolidone; (meth) acrylic acid esters having alkyl groups (having 1 to 22 carbon atoms) such as methyl(meth)acrylate, ethyl(meth)acrylate, n-butyl(meth)acrylate, n-octyl(meth)acrylate, isooctyl(meth)acrylate, 2-ethylhexyl(meth)acrylate, and n-decyl (meth)acrylate; (meth) acryl amides having a cyclic amide group such as N-(meth)acryloyl morpholine; esters of vinyl

alcohol having an alkyl group (having 1 to 22 carbon atoms) and aliphatic acid, such as vinyl carboxylate, vinyl propionate, vinyl pivalate, vinyl caproate, vinyl octylate, vinyl caprylate, vinyl caprinate, vinyl laurate, vinyl myristate, vinyl palmitate and vinyl stearate; and styrene-based monomers such as styrene, 2-methyl styrene and $\alpha$-methyl styrene dimer.

With respect to (C) the monomer having a cationic group, examples thereof include: (meth)acrylic acid esters or (meth)acryl amides having a dialkyl amino group having 2 to 44 carbon atoms in total, such as dimethyl aminoethyl (meth)acrylate, diethyl aminoethyl(meth)acrylate, dipropyl aminoethyl(meth)acrylate, diisopropyl aminoethyl(meth)acrylate, dibutyl aminoethyl(meth)acrylate, diisobutyl aminoethyl(meth)acrylate, di-t-butyl aminoethyl(meth)acrylate, dimethyl aminopropyl(meth)acryl amide, diethyl aminopropyl(meth)acryl amide, dipropyl aminopropyl(meth)acryl amide, diisopropyl aminopropyl(meth)acrylamide, dibutylaminopropyl(meth)acryl amide, diisobutyl aminopropyl (meth) acryl amide and di-t-butyl aminopropyl(meth)acryl amide; styrene having a dialkyl amino group having 2 to 44 carbon atoms in total such as dimethyl aminostyrene and dimethyl aminomethyl styrene; vinyl pyridine such as 2- or 4-vinyl pyridine; N-vinyl heterocyclic compounds such as N-vinyl imidazole; vinyl ethers such as aminoethyl vinyl ether, dimethyl aminoethyl vinyl ether; acid neutralized matters or quaternary compounds of a monomer having these amino groups; diallyl-type quaternary ammonium salts such as dimethyldiallyl ammonium chloride and diethyldiallyl ammonium chloride; and monomers such as vinyl monomers having a betaine structure, such as N-(3-sulfopropyl)-N-(meth) acryloyloxyethyl-N,N-dimethyl ammonium betaine, N-(3-sulfopropyl)-N-(meth)acryloylamidepropyl-N,N-dimethyl ammonium betaine, N-(3-carboxymethyl)-N-(meth) acryloylamidepropyl-N,N-dimethyl ammonium betaine and N-carboxymethyl -N-(meth)acryloyloxyethyl-N,N-dimethyl ammonium betaine.

With respect to acids that are preferably used for obtaining the above-mentioned acid-neutralized matters, examples thereof include hydrochloric acid, sulfuric acid, nitric acid, phosphoric acid or organic acids having 1 to 22 carbon atoms in total, such as acetic acid, formic acid, maleic acid, fumaric acid, citric acid, tartaric acid, adipic acid, sulfamic acid, toluene sulfonic acid, lactic acid, pyrrolidone-2-carboxylic acid, succinic acid, propionic acid or glycolic acid. With respect to preferable quaternarizing agents used for obtaining the above-mentioned quaternarized matters, halogenated alkyls (having 1 to 22 carbon atoms), halogenated benzyl, alkyl (1 to 18 carbon atoms) or aryl (6 to 24 carbon atoms) sulfonic acid or sulfuric dialkyls (2 to 8 carbon atoms in total) may be used, and among these, halogenated alkyls having 1 to 8 carbon atoms, such as methyl chloride, ethyl chloride, methyl bromide and methyl iodide, and dialkyl sulfates, such as dimethyl sulfate, diethyl sulfate and di-n-propyl sulfate, are preferably used.

Among these, with respect to monomers having copolymerizable polymer chain 1, of monomers (A) to (C), those having a repetitive structure derived from (A) and (C) monomers are preferably used; those having a repetitive structure derived from (A) monomers are more preferably used; those having a repetitive structure derived from one kind or more carboxylic acid-based monomers having a polymerizable unsaturated group, selected from the group consisting of (meth)acrylic acid, crotonic acid, itaconic acid, maleic acid and/or salts thereof are more preferably used; and those having a repetitive structure derived from (meth) acrylic acid and/or salts thereof are most preferably used.

The rate of a monomer having a phosphorous-based acid residue in the monomer forming polymer chain 1 is preferably set to 20 to 100 % by weight, more preferably, 50 to 100 % by weight, most preferably, 70 to 100 % by weight, in order to provide a desirable adsorbing property to a tooth.

[Polymer chain 2]

Referring to "Organic conceptual drawing-Basics and Applications-(written by Yoshio Kohda, first edition published by Sankyo Publishing Co., Ltd. on May 10, 1984)", the concept that a polymer chain is hydrophilic is defined by the fact that the ratio [I/O] between inorganic (I) and (O) organic polymer chains is not less than 0.6, preferably, not less than 1.0, more preferably, not less than 1.5.

With respect to hydrophilic polymer chain 2, polymer chains selected from the following groups of monomers (i) to (xii) are preferably used.

(i) (meth)acryl amides, (ii) alkyl (1 to 3 carbon atoms)(meth)acryl amides such as N-methyl(meth)acryl amide, N-ethyl(meth)acryl amide, N-n-propyl(meth)acryl amide and N-isopropyl(meth)acryl amide, (iii) dialkyl (2 to 8 carbon atoms in total)(meth)acryl amides, such as N,N-dimethyl(meth)acryl amide and N,N-diethyl(meth)acryl amide, (iv) (meth)acrylic acid esters or (meth)acyl amides having a hydroxy alkyl group (1 to 3 carbon atoms), (v) (meth)acrylic acids and salts thereof, (vi) monomers having a sulfonic acid group or salts thereof, such as styrene sulfonate, acryl amide propane sulfonate (AMPS) and methacryl sulfonate, (vii) amine-based monomers such as ethylene imine and allyl amine, (viii) alkylene oxides such as ethylene oxide and propylene oxide, (ix) monomers having a hydroxy group such as vinyl alcohol and allyl alcohol, (x) vinyl pyrrolidone, (xi) epichlorohydrin, and (xii) glycidyl ether and alkyl (1 to 3 carbon atoms) ethers thereof. With respect to the salts thereof, examples thereof include ammonium salts, alkali metal salts and alkali earth metal salts.

The hydrophilic polymer chain may be copolymerized with another monomer that is copolymerizable with the above-mentioned monomers, that is, for example, the monomers (A) to (C) described in the section of polymer chain

1 and the like, and in order to maintain its hydrophilic property, the ratio of the constituent unit selected from the group of monomers (i) to (xii) is preferably set to 70 to 100 % by weight, more preferably, 80 to 100 % by weight, most preferably, 90 to 100 % by weight, by far the most preferably, 100 % by weight, in the polymer chain 2.

In particular, among these, a single polymer, such as poly(meth)acryl amide, poly(meth)acrylic acid and salts thereof, polyethylene imine, polyallyl amine, polyoxyalkylene, polyvinyl alcohol, polyvinyl pyrrolidone, polyepichlorohydrin, polyglycidyl ether and alkyl (1 to 3 carbon atoms) ethers thereof, and polyoxyalkylene, which is easily produced, is more preferably used. With respect to the polyoxyalkylene, any one of polyoxyethylene, polyoxypropylene and random or block copolymers thereof may be used, and polyoxyethylene is more preferably used.
In particular, oral-use polymers represented by the following formula (III) are preferably used:

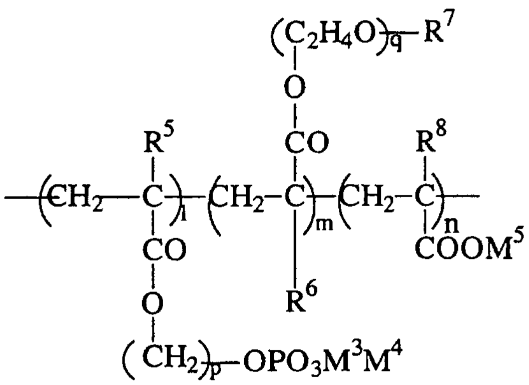

(III)

(wherein, l, m and n are set in the range of l : m : n = 1 to 95 : 5 to 80 : 0 to 94, and 0. 25 ≤ (l + n) / m ≤ 19, in mole % (l + m + n = 100) ; p represents a number of 1 to 22 and q represents a number of 1 to 200 in the number-average value; $R^5$, $R^6$, $R^7$ and $R^8$ each independently represent a hydrocarbon group that may be substituted by a hydrogen atom or a fluorine atom; $M^3$, $M^4$ and $M^5$ independently represent a hydrogen atom, metal, ammonium, alkyl or alkenyl ammonium having 1 to 22 carbon atoms in total, alkyl or alkenyl substituted pyridinium having 1 to 22 carbon atoms, alkanol ammonium having 1 to 22 carbon atoms in total, or basic amino acid residue.)

In formula (III), preferably, each of $R^5$, $R^6$, $R^7$ and $R^8$ may be substituted by a fluorine atom. In the case where a hydrocarbon group is used, a methyl group, an ethyl group and the like are listed, and in particular, a methyl group is preferably used. Moreover, in the case where each of $M^3$, $M^4$ and $M^5$ is metal, examples thereof include alkali metal, alkali earth metal and the like, and alkali metal is more preferably used. In the case where each of $M^3$, $M^4$ and $M^5$ is alkanol ammonium having 1 to 22 carbon atoms in total, preferable examples thereof include monoethanol ammonium, diethanol ammonium and triethanol ammonium, and when prepared as a basic amino acid residue, these are preferably arginine, lysine, histidine, ornithine and the like. More preferably, each of $M^3$, $M^4$ and $M^5$ is a hydrogen atom.

The mole % ratio of constituent monomers of the compound represented by formula (III) is set in the range of l : m : n = 1 to 95 : 5 to 80 : 0 to 94 (here, l + m + n = 100, and n may be 0), preferably, l : m : n = 1 to 92 : 8 to 66 : 0 to 91, more preferably, l : m : n = 1 to 90 : 10 to 20 : 0 to 89, and an inequality, 0. 25 ≤ (l + n) / m ≤ 19, is preferably satisfied, and from the viewpoint of bacteria adsorption inhibition to a tooth, this is set to 0.5 < (l + n) / m < 12, more preferably, 4 ≤ (l + n) / m ≤ 9. Here, each of l, m and n is an average value. Here, p is preferably set to 2 to 14, and from the viewpoint of adsorption inhibition to teeth, this is more preferably set to 8 to 12. Here, q is preferably set to 10 to 200 in the number average, more preferably, 20 to 200, most preferably, 40 to 150, by far the most preferably, 100 to 150.

[Synthesis of polymer]

The synthesizing method of the polymer of the present invention is not particularly limited, and any one of conventional methods may be selected. Among these, the following methods are preferably used: a method (macroazo initiator method) in which a vinyl-based monomer or the like is polymerized by using a macroazo initiator having an azo group in the polymer chain, a method (macromonomer method) in which a compound having a polymerizable group at one end of the polymer chain is used, a method (chain transfer method) in which a monomer is further radical-polymerized in the presence of a polymer so that a polymer chain, newly generated, is connected to a polymer chain that is allowed to preliminarily coexist through a chain transfer reaction and a method in which a polymer terminal of another kind of polymer is allowed to react with a functional group in a polymer chain and connected thereto. Among these methods, the following preparation method 1 or 2 is more preferably used, and preparation method 2 is most

preferably used.

Preparation method 1: a method in which a monomer represented by the above-mentioned formula (IV) and another vinyl monomer, if necessary, are radical-polymerized to obtain a block copolymer, by using a polyethylene glycol macroazo initiator.

Preparation method 2: a method in which a monomer represented by the above-mentioned formula (IV), a polyalkylene glycol mono(meth)acrylic acid ester represented by the following formula (V) and another vinyl monomer, if necessary, are polymerized to obtain a graft polymer.

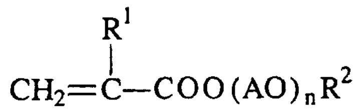

(V)

[wherein, $R^1$, $R^2$, AO and s are used in the same definitions as the aforementioned formulas.]

In the case of preparation method 2, a monomer represented by the above-mentioned general formula (IV) and a polyalkylene glycol mono(meth)acrylic acid ester represented by the above-mentioned genearl formula (V) are used, and in the presence of a radical polymerization initiator, these are polymerized through a known polymerization method, that is, a bulk polymerization method, a solution polymerization method, a suspension polymerization method, an emulsion polymerization method or the like to produce the target polymer, and in particular, the solution polymerization method is preferably used.

In this case, the rate of the monomer represented by the above-mentioned general formula (IV) and the polyalkylene glycol mono(meth)acrylic acid ester represented by the above-mentioned general formula (V) is preferably set in the range of (IV)/(V) (weight ratio) = 10/90 to 90/10, more preferably, 10/90 to 50/50, most preferably, 20/80 to 40/60.

In the case where a monomer selected from the above-mentioned (A) to (C) is further copolymerized with this, with respect to 100 parts by weight of the monomer represented by general formula (IV), the monomer selected from the group of (A) to (C) is preferably set to 5 to 50 parts by weight, more preferably, 5 to 30 parts by weight.

Although not particularly limited, the solvent to be used in the polymerization is preferably selected from the group consisting of water, alcohol, ketone and ether having not more than 3 carbon atoms, and water and alcohol having not more than 3 carbon atoms are more preferably used. These may be mixed at a desired rate and used. Alternatively, no solvent is used. With respect to alcohol having not more than 3 carbon atoms, examples thereof include methanol, ethanol, propanol and isopropanol; with respect to the ketones, examples thereof include acetone and methylethyl ketone; and with respect to the ethers, example thereof include tetrahydrofran, glyme, diglyme and dioxane, and in particular, ethanol, propanol and isopropanol are preferably used.

With respect to the polymerization initiator, although not particularly limited as long as it is dissolved in a solvent, preferable examples thereof include: persulfates such as ammonium persulfate, sodium persulfate and potassium persulfate; azo compounds such as 2,2'-azobis(2,4-dimethylvaleronitrile) and 2,2'-azobis(2-methylpropylene amidine) hydrochloride; and organic peroxides such as lauroyl peroxide. Alternatively, these may be combined with a reducing agent, and used as a redox-based initiator. The used amount of the polymerization initiator is preferably set in the range of 2.5 to 10 % by weight with respect to the monomer.

In order to control the molecular weight, a chain transfer agent such as mercaptoethanol may be used in combination. The used amount of the chain transfer agent is preferably set in the range of 0.5 to 10 mole % with respect to the monomer.

Although changed depending on the kinds of the polymerization initiator, the polymerization temperature is preferably set in the range of 60 to 95°C, and although changed depending on the kinds of monomers, polymerization initiators and reaction temperatures, the polymerization time is preferably set in the range of 1 to 12 hours.

Each of these monomers may be copolymerized as free acid, or may be neutralized by a salt such as sodium and potassium, and then polymerized. Alternatively, after the polymerization, it may be neutralized and used.

[Bacteria adsorption inhibitor and composition thereof]

The bacteria adsorption inhibitor made from the oral-use polymer of the present invention makes it possible to effectively inhibit adsorption of bacteria onto the surface of a tooth. With respect to the composition containing the bacteria adsorption inhibitor of the present invention, water, methanol, ethanol, isopropanol or the like may be used as a carrier. In this case, in the composition, the content of the bacteria adsorption inhibitor is preferably set in the range of 0.001 to 10 % by weight, more preferably, 0.1 to 5 % by weight, most preferably, 0.5 to 3 % by weight; thus,

this agent is easily applied.

The bacteria adsorption inhibiting property of the bacteria adsorption inhibitor of the present invention is indicated by a bacteria adsorption inhibitor value that is defined by the following formula (1), and under a concentration of not more than 1 % by weight of the oral-use polymer, this bacterial adsorption inhibitor value is preferably set to not less than 90, more preferably, not less than 95. The subject bacteria are preferably prepared as intraoral bacterial, more preferably, S. Mutans bacteria.

$$\text{Bacteria adsorption inhibitor value} = \frac{A - X}{A - B} \times 100 \qquad (1)$$

[wherein, A represents the number of bacteria that have adhered to a hydroxy apatite plate subjected to a saliva treatment ($\times 10^7$ cells/$cm^2$), B represents the number of bacteria that have adhered to a hydroxy apatite plate that was not subjected to the saliva treatment ($\times 10^7$ cells/$cm^2$), and X represents the number of bacterial that have adhered to a hydroxy apatite plate that was treated by each of oral-use polymers having % by weight shown in Table 1, and then subjected to the saliva treatment.]

In other words, a difference between the number of bacteria (A) adhered to the hydroxy apatite plate subjected to the saliva treatment and the number of bacteria (B) adhered to the hydroxy apatite plate that was not subjected to the saliva treatment is set as a reference value (100), and a difference between (A) and the number of bacteria (X) adhered to the hydroxy apatite plate that was treated by each of oral-use polymers having % by weight shown in Table 1, and then subjected to the saliva treatment was calculated by using the formula (1) ; thus, the bacteria adsorption inhibitor value was found.

[Bacterial-plaque-formation suppresser and the composition thereof]

The bacterial-plaque-formation suppressor made from the oral-use polymer of the present invention effectively suppresses formation of bacterial plaque onto the surface of a tooth. With respect to the composition containing the bacterial-plaque-formation suppresser of the present invention, water, methanol, ethanol, isopropanol or the like may be used as a carrier. In this case, the content of the bacterial-plaque-formation suppresser is preferably set in the range of 0.001 to 10 % by weight, more preferably, 0.1 to 5 % by weight, most preferably, 0.5 to 3 % by weight; thus, this agent is easily administered.

With respect to the dose of the bacterial-plaque-formation suppresser of the present invention per adult (60 kg), the oral-use polymer of the present invention is preferably set to 0.01 to 5 g/day, more preferably, 0.1 to 2.5 g/day, on a compound equivalent basis.

When the bacterial-plaque-formation suppresser of the present invention is administered to an infant or the like that has not been subjected to deposition of intraoral bacteria, it exerts effects for preliminarily preventing bacterial infection to the oral cavity.

[Oral-use composition]

The oral-use composition of the present invention containing the oral-use polymer of the present invention further contains water and/or lower alcohol having 1 to 5 carbon atoms as a carrier. Here, with respect to the lower alcohol, a straight-chain or branched-chain saturated alcohol having 1 to 5 carbon atoms is preferably used, and ethanol and isopropyl alcohol are more preferably used; in particular, ethanol is most preferably used. Here, two or more kinds selected from the group consisting of water and lower alcohols may be used in combination.

The content of the polymer of the present invention in the oral-use composition is preferably from 0.001 to 20 % by weight, more preferably, from 0.1 to 10 % by weight, most preferably, from 0.1 to 5 % by weight, by far the most preferably, from 0.5 to 3 % by weight. The content of water and/or lower alcohol is preferably from 0.1 to 99.9 % by weight, more preferably, from 5 to 80 % by weight.

From the viewpoint of bacterial-plaque inhibition, the oral-use composition of the present invention preferably contains a bactericide at a content from 0.1 to 5 % by weight, preferably, from 0.01 to 1 % by weight, with respect to the entire composition. With respect to the bactericide, examples thereof include: benzetonium chloride, chlorhexidine hydrochloride, trichlosan, cetylpyridinium chloride, isopropyl methylphenol, decalinium chloride and alkyl diaminoethyl glycine hydrochloride.

The oral-use composition of the present invention may appropriately contain components that are normally used for oral-use compositions, such as a surfactant, an abrasive, a sweetener, a perfume, a preservative, a skin treatment agent, a moistening agent and a binder, in a range so as not to impair the effects of the present invention.

With respect to the sweetener, preferable examples thereof include oligosaccharide or sugar alcohols, such as oxyoligosaccharide, fructooligosaccharide, galactooligosaccharide, soybean oligosaccharide, isomaltooligosaccharide, milk-well oligosaccharide, lactulose, raffinose, trehalose, glucosyl sucrose, maltosyl sucrose, palatinose, maltitol, erythritol, reduced palatinose and xylitol. In addition to these, the oral-use composition may preferably contain an enzyme inhibitor, a circulation accelerator, an anti-inflammatory agent, a hemostatic agent, a painkiller, an antihistaminic agent, and medicated agents, such as plant extracts, that exert the above-mentioned functions. With respect to the enzyme inhibitor, polyphenols (glucosyl transferase inhibiting function) may be used; with respect to the circulation accelerator, examples thereof include vitamin E, nicotinic acid d1-α-tocopherol and sodium chloride; with respect to the anti-inflammatory agent, examples thereof include: allantoin, β-glycyrrhizinic acid, glycyrrhizinic acid, epidihydrocholesterin, dihydrocholesterol, ε-amino caproic acid, hinokitiol, lysozyme chloride, indomethacin and ibuprofen; with respect to the hemostatic agent, examples thereof include: tranexamic acid, thrombin, ascorbic acid and rutinic acid; with respect to the painkiller, examples thereof include: aspirin, acetoaminophen and methyl salicylate; with respect to the antihistaminic acid, cimetidine, chlorphenylamine maleate, diphenehydramine hydrochloride and promethazine hydrochloride; with respect to the plant extract, examples thereof include: fennel extract, turmeric extract, scutellaria root extract, Hypericum erectum extract, chamomile extract, Sasa albo-marginata extract, Labiatae extract, sage extract, clove extract, ginseng extract, witch-hazel extract, fucus extract, horse chestnut extract, peach leave extract, rosemary extract, and eucalyptus extract. One or more kinds of these medicated agents may be used, and the content thereof is preferably from 0.1 to 5 % by weight, preferably, from 0.01 to 1 % by weight in the entire composition. In particular, by using a bactericide in combination, the bacterial plaque formation suppressing effect is increased so that the composition is effectively used as an oral-use composition; moreover, by using an anti-inflammatory agent in combination, it is possible to form an oral-use composition in which preventing effects against periodontal diseases are improved.

The oral-use composition of the present invention is manufactured through a normal method. Moreover, the composition is applied to the oral cavity through a normal method, and it is also used upon massaging the gums.

The oral-use composition of the present invention may be used as various oral agents such as tooth paste, tooth powder, water toothpaste, liquid-state toothpaste, liquid toothpaste, lubricating toothpaste, mouth-rinsing agent, mouth wash, mouth spray, tooth coating agent, false-tooth coating agent, false-tooth washing agent and chewing gums. In particular, it is preferably used as tooth paste.

The oral-use polymer of the present invention serves as a bacteria-adsorption inhibitor having a high bacteria-adsorption inhibiting effect so that it inhibits adsorption of saliva protein onto the tooth surface, which causes bacteria adsorption, thereby inhibiting and suppressing adsorption and infection of bacteria, and also serves as a bacterial-plaque-formation suppresser agent for suppressing formation of bacterial plaque, and the oral-use composition containing the oral-use polymer of the present invention preliminarily prevents oral cavity diseases caused by bacterial plaque.

The measuring conditions of a weight-average molecular weight through gel permeation chromatography (GPC) are shown below:

GPC measuring conditions
Column G4000PWXL + G2500PWXL (made by Tosoh Corporation)
Eluting solvent 0.2N phosphoric acid buffer solution : acetonitrile = 9 : 1 (capacity ratio)
(The phosphoric acid buffer solution was prepared by dissolving $KH_2PO_4$ and $Na_2HPO_4$ to form a 0.2N solution with the pH being set to 7.)
Flow rate 1 ml/min
Column temperature 40°C
Sample 5 mg/ml
Detection RI
Conversion molecular weight polyethylene glycol

BRIEF DESCRIPTION OF THE DRAWINGS

Fig. 1 shows results of proton NMR of polymer 1 obtained in synthesis example 1; and
Fig. 2 shows results of proton NMR of polymer 2 obtained in synthesis example 2.

Examples

The following description will discuss synthesis examples and examples. Here, "%" refers to "weight %", unless otherwise defined.

Synthesis example 1 (Synthesis of polymer 1)

To a four-neck separable flask (300 ml) were loaded 16.0 g of ion exchanged water, 20. 9 g (3.9 mmol) of methacrylic acid ester of polyethylene glycol (average addition mole number of oxyethylene group: 120, I/O value: 2), 7.4 g (22.9 mmol) of methacryloyloxydecyl phosphoric acid, 1.2 g (14.5 mmol) of methacrylic acid, 0.5 g of 2-mercaptoethanol, 1.2 g of ammonium persulfate and 8.8 g of isopropyl alcohol to form an even solution. After having been subjected to a nitrogen-substituting process, this was heated to 95°C so that a polymerizing process was carried out for 6 hours. After having been cooled off, this was put into a cellulose tube and subjected to a film dialysis for three days. Then, this was freeze-dried to obtain 25.1 g of colorless powder (polymer 1) .

The resulting product was prepared as a 10 % heavy water solution, and subjected to measurements of proton NMR (Fig. 1) , the following protons were confirmed: protons derived from a methylene group joined to a carboxyl group at δ= 4.1; protons derived from methylene of a polyoxy ethylene chain at δ= 3.6 to 3.8; protons derived from methylene in a polymer main chain at δ= 1.6 to 1.8; protons derived from methylene between a carboxylic ester and a phosphoric acid group in methacryloyl oxydecyl phosphoric acid at δ= 1.4; and protons derived from a methyl group joined to the main chain at δ= 0.8 to 1.2; thus, it was confirmed that the compound is a copolymer of a methacrylic acid ester of polyethylene glycol, methacryloyloxydecyl phosphoric acid and methacrylic acid. The weight-average molecular weight was found to be 31000 through gel permeation chromatography (GPC: polyethylene glycol conversion). The molecular weight of polymer chain 1 was found to be 8000 and the molecular weight of polymer chain 2 was found to be 5000 based upon NMR and GPC.

Synthesis example 2 (Synthesis of polymer 2)

The same processes as synthesis example 1 of example 1 were carried out except that no methacrylic acid was added to prepare 25.5 g of colorless powder (polymer 2).

The resulting product was prepared as a 10 % heavy water solution, and subjected to measurements of proton NMR (Fig. 2) , the following protons were confirmed: protons derived from a methylene group joined to a carboxyl group at δ= 3.9; protons derived from methylene of a polyoxy ethylene chain at δ= 3.6 to 3.8; protons derived from methylene in a polymer main chain at δ= 1.6 to 1.8; protons derived from methylene between a carboxylic ester and a phosphoric acid group in methacryloyl oxydecyl phosphoric acid at δ= 1.4; and protons derived from a methyl group joined to the main chain at δ= 0.8 to 1.2; thus, it was confirmed that the compound is a copolymer of a methacrylic acid ester of polyethylene glycol and methacryloyloxydecyl phosphoric acid. The weight-average molecular weight was found to be 48000. The molecular weight of polymer chain 1 was found to be 12000 and the molecular weight of polymer chain 2 was found to be 5000 based upon NMR and GPC.

Synthesis example 3 (Synthesis of polymer 3)

To a four-neck separable flask (500 mL) were loaded 68 g of ion exchanged water, and this was heated to 80°C, while being nitrogen-substituted. In a separate manner, to a beaker (300 mL) were loaded 111.0 g of ion exchanged water, 49.7 g (9.3 mmol) of methacrylic acid ester of polyethylene glycol (average addition mole number of oxyethylene group: 120, I/O value: 2), 17.5 g (54.3 mmol) of methacryloyloxydecyl phosphoric acid, 3.0 g (34.8 mmol) of methacrylic acid, 6.6 g of 2-mercaptoethanol, 0.97 g of ammonium persulfate and 24.1 g of ethanol to form an even solution. This was dripped in the above-mentioned four-neck separable flask in four hours. Thereafter, to this was further dripped 4.8 g of a 10 % ammonium persulfate aqueous solution in one hour. After having been matured for 2 hours, to this were added 12 g of a 48 % sodium hydroxide aqueous solution and 16.5 g of a 35 % hydrogen peroxide aqueous solution, and this was matured for one hour. After having been cooled off, the total amount of this was put into a cellulose tube and subjected to a film dialysis for three days. Then, this was freeze-dried to obtain 27.5 g of colorless powder. The weight-average molecular weight was found to be 16,000 through GPC. The molecular weight of polymer chain 1 was found to be 2,400 and the molecular weight of polymer chain 2 was found to be 5,000 based upon NMR and GPC.

Synthesis example 4 (Synthesis of polymer 4)

To a four-neck separable flask (500 mL) were loaded 102 g of ion exchanged water, and this was heated to 80°C, while being nitrogen-substituted. In a separate manner, to a beaker (300 mL) were loaded 166.0 g of ion exchanged water, 74.6 g (13.9 mmol) of methacrylic acid ester of polyethylene glycol (average addition mole number of oxyethylene group: 120, I/O value: 2), 26.3 g (81.7 mmol) of methacryloyloxydecyl phosphoric acid, 4.5 g (4.3 mmol) of methacrylic acid, 9.9 g of 2-mercaptoethanol, 1.5 g of ammonium persulfate and 26.3 g of ethanol to form an even solution. This was dripped in the above-mentioned four-neck separable flask in four hours. Thereafter, to this was

further dripped 7.4 g of a 10 % ammonium persulfate aqueous solution in one hour. After having been matured for 2 hours, to this were further added 18 g of a 48 % sodium hydroxide aqueous solution and 24.8 g of a 35 % hydrogen peroxide aqueous solution, and this was matured for one hour. After having been cooled off, the total amount of this was put into a cellulose tube and subj ected to a film dialysis for three days. Then, this was freeze-dried to obtain 56.2 g of colorless powder. The weight-average molecular weight was found to be 15,000 through GPC. The molecular weight of polymer chain 1 was found to be 3, 600 and the molecular weight of polymer chain 2 was found to be 5,000 based upon NMR and GPC.

Comparative synthesis example 1 (Synthesis of comparative polymer 1)

To a four-neck separable flask (300 ml) were loaded 68 g of ion exchanged water, and this was heated to 85°C, while being nitrogen-substituted. In a separate manner, to a beaker (200 mL) were loaded 50 g (9.3 mmol) of methacrylic acid ester of polyethylene glycol (average addition mole number of oxyethylene group: 120, I/O value: 2), 1.5 g (17.3 mmol) of methacrylic acid, 0.15 g of 2-mercaptoethanol, 0.55 g of ammonium persulfate and 110 g of ion exchanged water to form an even solution. This was dripped in the above-mentioned four-neck separable flask in four hours. Thereafter, to this was further dripped 2.5 g of a 10 % ammonium persulfate aqueous solution in one hour. After having been matured for 2 hours, to this were further added 1.4 g of a 48 % sodium hydroxide aqueous solution and 16.5 g of a 35 % hydrogen peroxide aqueous solution, and this was matured for one hour. After having been cooled off, the total amount of this was put into a cellulose tube and subjected to a film dialysis for three days. Then, this was freeze-dried to obtain 48.0 g of colorless powder (comparative polymer 1). The weight-average molecular weight was found to be 80,000 through GPC. The molecular weight of the main chain was found to be 2,400 and the molecular weight of the side chain was found to be 5, 000 based upon NMR and GPC.

Comparative synthesis example 2 (Synthesis of comparative polymer 2)

To a four-neck separable flask (300 ml) were loaded 30.0 g of ethanol, 20.0 g (62.1 mmol) of methacryloyloxydecyl phosphoric acid and 0.5 g (2.0 mmol) of 2,2'-azobis(2,4-dimethyl valeronitrile) to form an even solution. After having been nitrogen-substituted, this was heated to 60°C so that a polymerizing process was carried out in 2.5 hours. After having been heated to 75°C, to this was dripped a solution prepared by dissolving 0.5 g (2.0 mmol) of 2,2'-azobis (2,4-dimethyl valeronitrile) in 6 g of ethanol in five minutes. After having been matured for 2.5 hours, this was cooled off, and an appropriate amount of water was added to this, and the resulting solution was put into a cellulose tube, and subjected to a film dialysis for three days. Then, this was freeze-dried to obtain 25.1 g of colorless powder (comparative polymer 2). The weight-average molecular weight was found to be 76,000 through GPC.

Example 1: Measurements on bacteria adsorption inhibiting property

Each of the polymers obtained through synthesis examples 1 to 4 and comparative synthesis examples 1 and 2, as well as each of comparative polymers shown in Table 1, was used as a bacteria adsorption inhibiting agent and the bacteria adsorption inhibiting property was measured by the following method.

<Measuring method for bacteria adsorption inhibiting property>

S.mutans mycobiont (10 μCi/mL), isolated from the human oral cavity, was inoculated on 10 mL of methylated [$^3$H]-thymidine Brainheart Infusion culture medium containing 0.2 % by weight of glucose (made by DIFCO Co., Ltd.), and this was subj ected to an anaerobic culturing process at 37°C for 24 hours. This was washed with a buffer KCl solution (buffer solution of 1 mM phosphoric acid containing 50 mM of potassium chloride, 1 mM of magnesium chloride and 0.1 mM of magnesium chloride) three times, and then dispersed in a buffer potassium chloride solution containing 5 mg/mL of bovine serum albumin at a concentration of $1 \times 10^9$CFU/mL, thereby preparing a $^3$H labeled S.mutans solution.

A hydroxy apatite flat plate (made by Pentax Corporation) having a size of 1 cm × 1 cm × 2 cm was treated at 37°C for one hour by using a 1 mL of bacteria adsorption inhibitor aqueous solution having each concentration shown in Table 1. After having been washed with 2 mL of a buffer potassium chloride aqueous solution, this was treated in 0.5 mL of parotid-gland saliva sampled from a normal male subject at 4°C for one night. After having been washed with 2 mL of buffer potassium chloride solution twice, to this was added 0.5 mL of a buffer potassium chloride solution containing 5 mg/mL of bovine serum albumin and 0.5 mL of the above-mentioned $^3$H labeled S.mutans solution, and this was treated at 37°C for one hour. After having been washed with a buffer potassium chloride solution three times, the resulting hydroxy apatite flat plate was treated in 1 mL of 2 M/L sodium hydroxide at 70°C for one hour. After having been neutralized by 1 mL of 2N hydrochloric acid, the $^3$H radiation activity was measured by using a solution scintillation

counter so that the number of adsorbed bacteria (X) was obtained.

Here, it is supposed that the number of adsorbed bacteria is A when the same processes are carried out by using 1 mL of distilled water in place of the bacteria adsorption inhibitor solution having each concentration shown in Table 1. The resulting value was A = 2.5 ($\times 10^7$ cells/$cm^2$).

Moreover, it is supposed that the number of adsorbed bacteria is B when the same processes are carried out by using 1 mL of distilled water in place of the bacteria adsorption inhibitor solution having each concentration shown in Table 1, with 0.5 mL of a buffer potassium chloride solution being used in place of parotid-gland saliva. The resulting value was B = 0.52 ($\times 10^7$ cells/$cm^2$).

Based upon the values of A, B and X, the bacteria adsorption inhibiting value, defined by the above-mentioned formula (1), was found.

Table 1 shows results of measurements; and any of polymers 1 to 4 that are the bacteria adsorption inhibitors of the present invention show excellent bacteria adsorption inhibiting effects.

Table 1

| | p | q | l/m/n | Concentration (weight %) | Suppressing rate (%) |
|---|---|---|---|---|---|
| Polymer 1 | 10 | 120 | 68/13/19 | 1 | 96 |
| Polymer 2 | 10 | 120 | 86/14/0 | 1 | 90 |
| Polymer 3 | 10 | 120 | 31/16/53 | 0.5 | 97 |
| Polymer 4 | 10 | 120 | 77/15/8 | 0.5 | 95 |
| Comparative polymer 1 | – | 120 | – | 0.5 | 45 |
| Comparative polymer 2 | 10 | – | 100/0/0 | 0.5 | 24 |
| Polyacrylic acid (note 1) | – | – | – | 0.5 | 40 |
| Polyacrylic acid maleic acid (note 2) | – | – | – | 0.5 | 86 |
| Alginic acid (note 3) | – | – | – | 0.5 | 71 |
| Copolymer of dimethyldiacryl ammonium chloride/acrylic acid (note 4) | – | – | – | 0.5 | 5 |

(note 1) Average molecular weight 3000,000, made by Aldrich

(note 2) Average molecular weight 50000, Sokaran* CP-07, made by BASF

(note 3) 100 to 150 CP made by Wako Pure Chemical Industries, Ltd.

(note 4) Average molecular weight 1700,000; Merquat* 280, made by calgon Co.

Example 2 Bacterial infection suppressing effect

The following experiments were carried out in accordance with the method described by Ooshima T. et al.

(1992) Caries Res 26: 124-131. In other words, each of 24-day old male SD rats (Charles River Co., Ltd.) was allowed to freely take MF powder feed containing tetracycline and distilled water containing Penicillin G for three days, and Streptomycin resistant S. mutans (obtained by continuously raising these in a Mitis-salivarius nutrient agar through generations, wherein the concentration of Streptomycin was increased from 0 to 500 µg/mL step by step) were then inoculated in the oral cavity of the rat at a rate of $2 \times 10^9$CFU/rat for 5 days. During ten days including the S. mutans inoculation period, Diet 2000 feed (made by Oriental Yeast Co., Ltd.) was given to the rat, and the rat was allowed to freely take polymer 3 aqueous solution (1 %).

The lower jaw was taken out, and adhering bacteria were ultrasonic-dispersed on 4.5 mL of Brainheart Infusion culture medium (made by DIFCO Co., Ltd.). After having been diluted in the culture medium step by step, 100 µL of this was inoculated on a Mitis-salivarius agar medium containing 100 µg/mL of Streptomycin, and after having been subjected to an anaerobic culturing process for 24 hours, this was subjected to an aerobic culturing process for 24 hours. The number of grown-up colonies was measured so that the number of bacteria adhering to the lower jaw was calculated.

After 10 days of administration, the number of bacteria infected rats (Table 2) increased significantly in the group of (inoculated) distilled water due to bacterial inoculation in comparison with the non-inoculation group. In comparison with the distilled water group, bacterial infection was virtually inhibited completely in the rats in the polymer 3 group.

Table 2

| | Non-inoculation group (5 rats) | Distilled water group (5 rats) | Polymer 3 group(5 rats) |
|---|---|---|---|
| Number of infection bacteria (x $10^4$ CFU) | 0±0 | 1.2±0.2 | 0.01±0.01 |

Example 3 Bacterial-plaque-formation suppressing effects

Cutting teeth of 10 male ODU rats (Osaka Dental College) were completely cleaned, and the rats were divided into two groups. The test group were allowed to freely take polymer 3 aqueous solution (1 %), while the control group were allowed to freely take distilled water, and the rates of both of the groups (n = 5/group) were allowed to freely take MF powder feed (Oriental Yeast Co., Ltd.) containing 50 % of cane sugar. After 96 hours, bacterial plaque was dyed so that the plaque adhesion amount was measured based upon the area (length × width) of the plaque adhesion region.

After keeping the rats while allowing them to freely take solid-state feed and drinking water for 5 days, the groups were exchanged and subjected to the same tests.

With respect to the plaque adhesion amount (Table 3) 96 hours later, the plaque formation was suppressed by 63 % ($P < 0.0001$) in the ODC rats in the polymer 3 group in comparison with the control group. Although the amount of drunken water was significantly small in the polymer 3 group, there was no significant difference in the food consumption and the amount of weight increase.

Table 3

| | Control group (10 rats) | Polymer 3 group (10 rats) | Significance |
|---|---|---|---|
| Bacteria plaque adh esion area $mm^2$ | 4.8±2.0 | 1.8±1.9 | P<0.0001 |
| Amount of drunken w ater g/day | 33. 7±7. 6 | 24. 8±3. 1 | P<0. 01 |
| Food consumption g /day | 15.2±2.2 | 15.0±1.5 | N. S. |
| Amount of weight in crease g | 6.9±6.0 | 8.1±3.3 | N. S. |

Example 4 Calcium crystallization inhibiting effects

With respect to calcium crystallization inhibiting activity, measurements were carried out in accordance with the method described in the known publication (Hay DI et al. (1984) J. Dent. Res. 63: 857-863).

In other words, to 0.5 mL of a test sample dissolved in a measuring buffer (0.05 M imidazole-hydrochloric acid buffer solution containing 0.15 M sodium chloride and 0.03 M sodium azide: pH7) were added 2.5 mL of phosphoric acid solution (containing 0.011 M potassium dihydrogen phosphate, 0.056 M potassium chloride, 0.003 M sodium azide: pH6.8) and 0.5 mL of a calcium solution (prepared by dissolving 2.382 g of calcium carbonate in 20 mL of 3 M hydrochloric acid, and adjusted to 11 with distilled water) , and this was stirred at 37°C for 24 hours. This was filtered through a filter membrane of 0.45 µm, and calcium in the filtrate was quantity-measured by a Calcium E Test Wako

made by Wako Pure Chemical Industries, Ltd. so that the amount of crystallized calcium was calculated based upon the reduced amount of dissolved calcium; thus, the calcium crystallization inhibiting activity was calculated based upon the following expression:

$$\text{Calcium crystallization inhibiting activity} = \frac{\text{Quantity of crystallized calcium at the time of addition of test sample}}{\text{Quantity of crystallized calcium without test sample (control: measuring buffer)}} \times 100\ (\%)$$

Table 4

| Polymer 3 concentration(μg/mL) | Ca crystallization inhibiting activity(%) |
|---|---|
| 10 | -8.26±2.66 |
| 50 | 3.98±4.19 |
| 250 | 80.9±3.38 |
| 750 | 104±1.57 |

Prescription Example 1 Toothpaste agent

| | |
|---|---|
| Polymer 3 | 5 % by weight |
| Silica | 30 |
| Sorbitol | 30 |
| Sodium lauryl sulfate | 1.0 |
| Carboxymethyl cellulose | 1.0 |
| Saccharin | 0.1 |
| Perfume | proper amount |
| Water | Total 100 |

Prescription Example 2 Mouthwash agent

| | |
|---|---|
| Polymer 3 | 5 % by weight |
| Ethanol | 20 |
| Sodium pyrophosphate | 2.0 |
| Carboxymethyl cellulose | 1.0 |
| Saccharin | 0.1 |
| Perfume | proper amount |
| Water | Total 100 |

Both of the oral-use compositions in prescription examples 1 and 2 of the present invention exerted excellent bacterial-plaque-formation inhibiting effects.

Prescription Example 3: Toothpaste agent

Based upon the following prescription, a toothpaste was manufactured in accordance with a conventional method.

| | |
|---|---|
| Polymer 1 | 5.0 % by weight |
| Silica | 30.0 |
| Sorbitol | 30.0 |

(continued)

| | |
|---|---|
| Sodium lauryl sulfate | 1.0 |
| Carboxymethyl cellulose*1 | 1.0 |
| Saccharin | 0.1 |
| Benzetonium chloride | 0.5 |
| Perfume | 0.3 |
| Water | 32.1 |
| Total | 100 % |

*1: Carboxymethyl cellulose (Brand Name: Sunrose F20-HC, made by Nippon Paper Industries Co., Ltd.)

Prescription Example 4: Mouthwash agent

Based upon the following prescription, a mouthwash agent was manufactured in accordance with a conventional method.

| | |
|---|---|
| Polymer 2 | 5.0 % by weight |
| Ethnol | 20.0 |
| Sodium pyrophosphate | 2.0 |
| Saccharin | 0.1 |
| Hydrochloric acid chlorhexidine | 0.3 |
| Perfume | 0.3 |
| Water | 72.3 |
| Total | 100 % |

## Claims

**1.** An oral-use polymer comprising:

a polymer chain (hereinafter, referred to as polymer chain 1) containing a repetitive structure derived from a monomer having a residue obtained by removing one hydrogen atom from one kind or more of phosphorus-containing acids selected from the group consisting of phosphoric acid, polyphosphoric acid, phosphonic acid, polyphosphonic acid, phosphinic acid, partial esters thereof and salts thereof; and
a hydrophilic polymer chain (hereinafter, referred to as polymer chain 2).

**2.** The oral-use polymer according to claim 1, comprising a graft polymer having polymer chain 1 as a main chain with polymer chain 2 being a side chain.

**3.** The oral-use polymer according to claim 1 or 2, wherein polymer chain 2 is a polyoxyalkylene chain.

**4.** The oral-use polymer according to any one of claims 1 to 3, wherein the molecular weight ratio (polymer chain 1/polymer chain 2) of polymer chain 1 and polymer chain 2 is set in the range of 10/1 to 1/10.

**5.** The oral-use polymer according to any one of claims 1 to 4, wherein polymer chain 1 further comprises:

a repetitive structure derived from a carboxylic acid-based monomer having not less than one kind of polymerizable unsaturated group selected from the group consisting of (meth) acrylic acid, crotonic acid, itaconic acid, maleic acid and/or salts thereof.

**6.** The oral-use polymer according to any one of claims 1 to 5, comprising a repetitive structure represented by formula (I) and a repetitive structure represented by formula (II) :

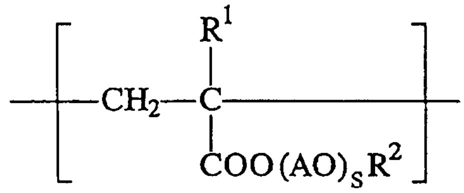

(I)

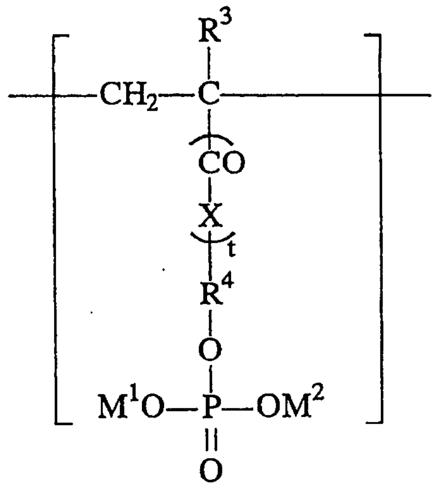

(II)

[whereins, $R^1$ and $R^3$, which may be the same or different from each other, represent a monovalent hydrocarbon group that may be substituted by a hydrogen atom or a fluorine atom, $R^2$ represents a monovalent hydrocarbon group that may have a hydrogen atom or a substituent, and $R^4$ represents a divalent hydrocarbon group. AO represents an oxyalkylene group having 2 to 3 carbon atoms, s represents a number of 1 to 200 in the number average value, and s-number of AO' s may be the same or different from each other. X represents -O- or -NH-, and t represents 0 or 1. $M^1$ and $M^2$, which may be the same or different from each other, represent a hydrogen atom, metal, ammonium, alkyl or alkenyl ammonium having 1 to 22 carbon atoms in total, alkyl or alkenyl substituted pyridinium having 1 to 22 carbon atoms, alkanol ammonium having 1 to 22 carbon atoms in total, or basic amino acid.]

**7.** An oral-use polymer represented by the following formula (III):

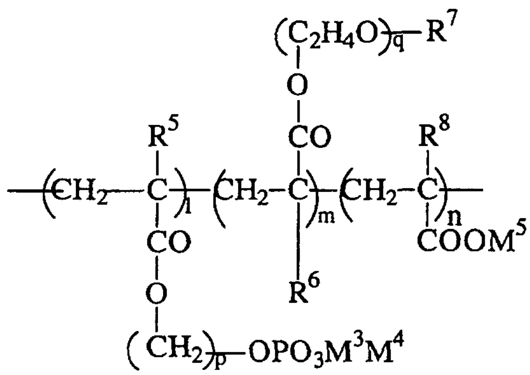

(III)

(wherein, l, m and n are set in the range of l : m : n = 1 to 95 : 5 to 80 : 0 to 94, and $0.25 \leq (l + n) / m \leq 19$, in mole % (l + m + n = 100) ; p represents a number of 1 to 22 and q represents a number of 1 to 200 in the number-average value; $R^5$, $R^6$, $R^7$ and $R^8$ each independently represent a monovalent hydrocarbon group that may be

substituted by a hydrogen atom or a fluorine atom; $M^3$, $M^4$ and $M^5$ independently represent a hydrogen atom, metal, ammonium, alkyl or alkenyl ammonium having 1 to 22 carbon atoms in total, alkyl or alkenyl substituted pyridinium having 1 to 22 carbon atoms, alkanol ammonium having 1 to 22 carbon atoms in total, or basic amino acid residue)

**8.** A bacteria adsorption inhibitor comprising the oral-use polymer described in any one of claims 1 to 7.

**9.** A bacterial-plaque-formation suppressor comprising the oral-use polymer described in any one of claims 1 to 7.

**10.** An oral-use composition comprising the oral-use polymer described in any one of claims 1 to 7.

**11.** An application of the oral-use polymer described in any one of claims 1 to 7 as a bacteria adsorption inhibitor or a bacterial-plaque-formation suppressor.

**12.** A method for applying the oral-use polymer described in any one of claims 1 to 7 to the oral cavity to inhibit adsorption of bacteria or to suppress formation of bacterial plaque.

**13.** An oral-use composition comprising the oral-use polymer described in any one of claims 1 to 7 and a medium.

Fig 1.

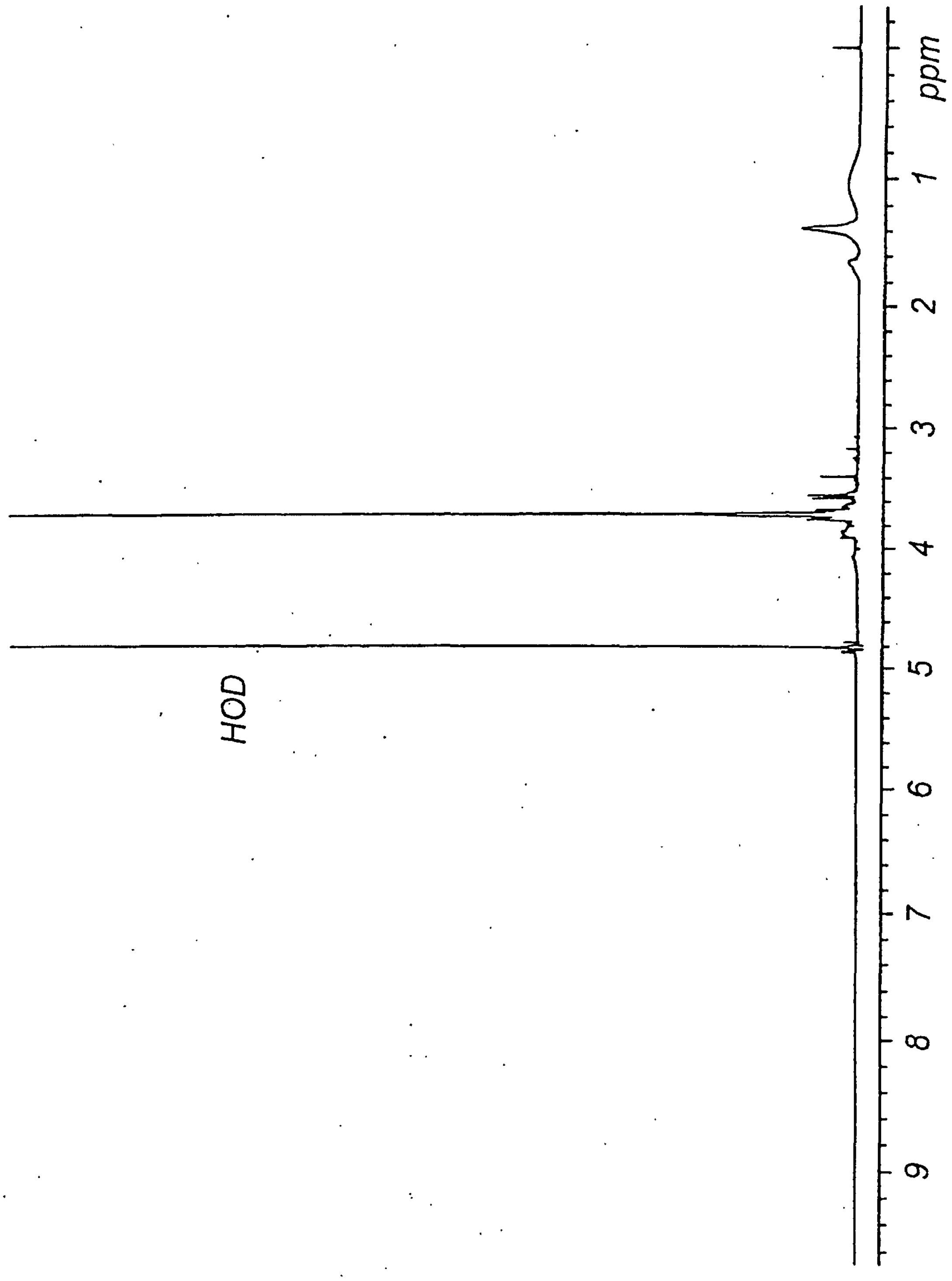

Fig 2

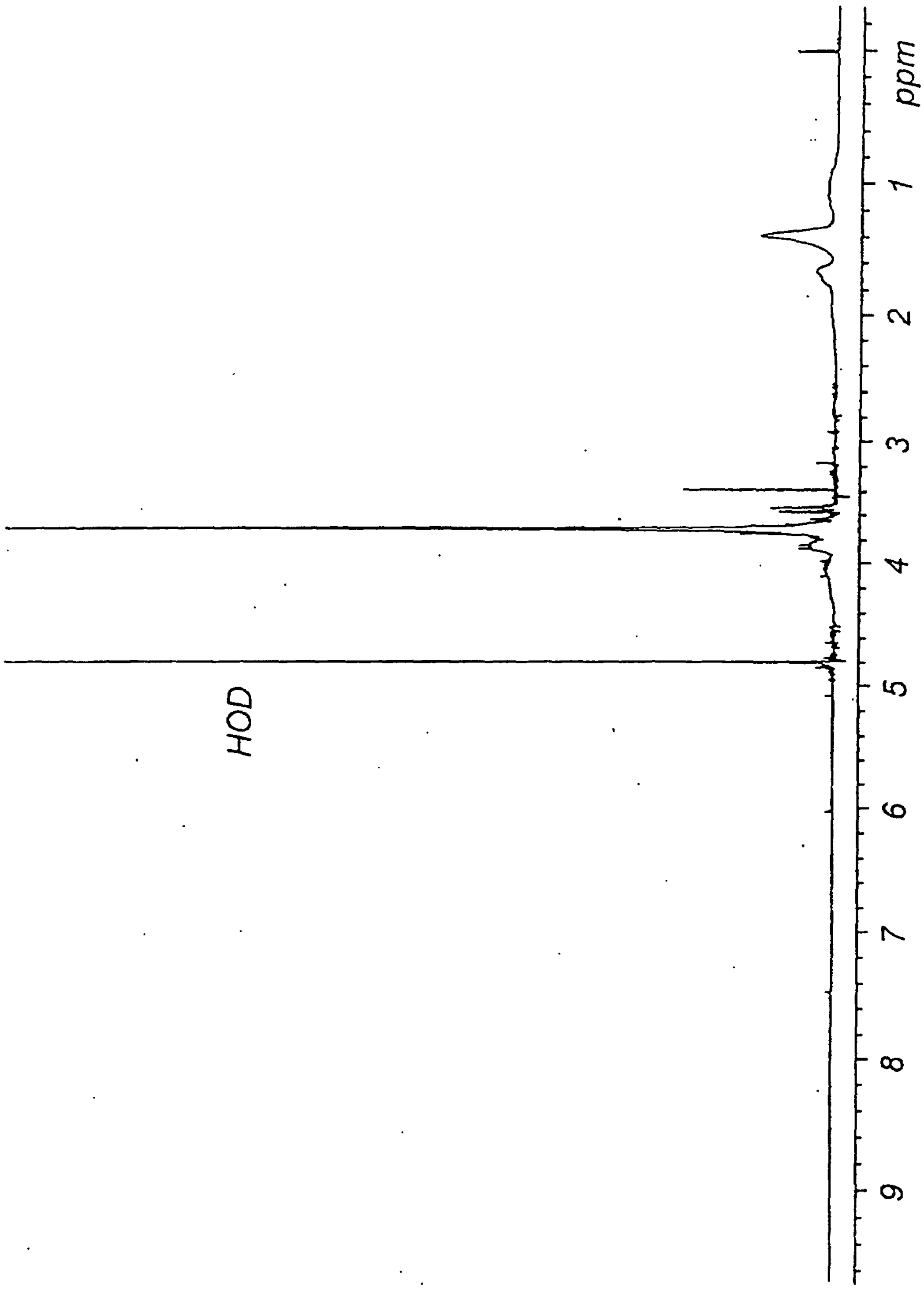

## INTERNATIONAL SEARCH REPORT

International application No.
PCT/JP02/07066

A. CLASSIFICATION OF SUBJECT MATTER
Int.Cl$^7$ C08F290/06, A61K7/16, A61K31/80, A61P1/02, A61P31/04, A61K47/32

According to International Patent Classification (IPC) or to both national classification and IPC

B. FIELDS SEARCHED

Minimum documentation searched (classification system followed by classification symbols)
Int.Cl$^7$ C08F290/00-290/14, C08F299/00-299/08, A61K7/16, A61K31/80, A61P1/02, A61P31/04, A61K47/32

Documentation searched other than minimum documentation to the extent that such documents are included in the fields searched

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X | JP 2000-327386 A (Taiheiyo Sement Corp.), 28 November, 2000 (28.11.00), Claims; examples (Family: none) | 1-7 |
| X | JP 62-240372 A (Denki Kagaku Kogyo Kabushiki Kaisha), 21 October, 1987 (21.10.87), Claims; examples (Family: none) | 1-7 |
| A | GB 2227660 A (Colgate-Palmolive Co.), 08 August, 1990 (08.08.90), Claims & GB 2227661 A & GB 2235201 A & GB 2235133 A & GB 2235133 A & GB 2259856 A & JP 2-288819 A | 8-13 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 15 October, 2002 (15.10.02) | 29 October, 2002 (29.10.02) |
| Name and mailing address of the ISA/ Japanese Patent Office | Authorized officer |
| Facsimile No. | Telephone No. |

Form PCT/ISA/210 (second sheet) (July 1998)

INTERNATIONAL SEARCH REPORT

International application No.
PCT/JP02/07066

C (Continuation). DOCUMENTS CONSIDERED TO BE RELEVANT

| Category* | Citation of document, with indication, where appropriate, of the relevant passages | Relevant to claim No. |
|---|---|---|
| | & JP 2-288820 A & JP 3-83910 A<br>& JP 3-83911 A & ZW 17289 A<br>& AU 4676889 A & AU 4676989 A<br>& AU 4677189 A & AU 4676689 A<br>& FI 896316 A & FI 896317 A<br>& FI 896318 A & FI 946119 A<br>& HU 52695 A & HU 54486 A<br>& HU 52694 A & ZM 5089 A<br>& IE 65326 A & IE 65326 B<br>& IE 65678 B & SE 8904179 A0<br>& SE 8904179 A & SE 8904180 A<br>& SE 8904181 A & LU 87652 A<br>& LU 87651 A & LU 87650 A<br>& DK 670989 A & DK 671089 A<br>& DK 671189 A & DK 671289 A<br>& IL 92691 A & IL 92692 A<br>& IL 92693 A & IL 92694 A<br>& IL 98606 A & IL 98607 A<br>& NO 895309 A & NO 895310 A<br>& NO 895311 A & IT 1237483 A<br>& IT 1237484 A & IT 1238355 A<br>& IT 1241168 A & IN 173330 A<br>& IN 173340 A & IN 173628 A<br>& IN 173759 A | |
| A | EP 467548 A1 (Colgate-Palmolive Co.),<br>22 January, 1992 (22.01.92),<br>Claims<br>& EP 469722 A2 & EP 579333 A2<br>& EP 579383 A1 & EP 584877 A2<br>& EP 696449 A2 & EP 743059 A2<br>& EP 525913 A1 & US 4894220 A1<br>& US 5032386 A1 & US 5037635 A1<br>& US 5037637 A1 & US 5043154 A1<br>& US 5080887 A1 & US 5135738 A1<br>& US 5156835 A1 & US 5167951 A1<br>& US 5178851 A1 & US 5180578 A1<br>& US 5188821 A1 & US 5192530 A1<br>& US 5192531 A1 & US 5202112 A1<br>& US 5234688 A1 & US 5256401 A1<br>& US 5260062 A1 & US 5273741 A1<br>& US 5279813 A1 & US 5288480 A1<br>& US 5292526 A1 & US 5294431 A1<br>& US 5296214 A1 & US 5300283 A1<br>& US 5312618 A1 & US 5334375 A1<br>& US 5344641 A1 & US 5385729 A1<br>& US 5424059 A1 & US 5453265 A1<br>& US 5466437 A1 & US 5496540 A1<br>& US 5531982 A1 & US 5538715 A1<br>& US 5575652 A1 & US 5686064 A1<br>& US 5728756 A1 & US 5776435 A<br>& JP 63-258404 A & JP 4-270210 A<br>& JP 5-105617 A & JP 5-194166 A<br>& JP 6-192060 A & JP 8-310968 A<br>& JP 3112914 B & AU 4158193 A | 8-13 |

Form PCT/ISA/210 (continuation of second sheet) (July 1998)

**INTERNATIONAL SEARCH REPORT**

International application No.
PCT/JP02/07066

C (Continuation). DOCUMENTS CONSIDERED TO BE RELEVANT

| Category* | Citation of document, with indication, where appropriate, of the relevant passages | Relevant to claim No. |
|---|---|---|
| | & AU 4676889 A & AU 4676989 A<br>& AU 2451992 A & AU 4677189 A<br>& AU 4676689 A & AU 5199993 A<br>& AU 2076192 A & AU 1017588 A<br>& AU 7423591 A & AU 7423691 A<br>& AU 7423791 A & AU 7423891 A<br>& AU 7423991 A & AU 4005893 A<br>& AU 7940091 A & AU 2326295 A<br>& AU 5231396 A & AU 7945291 A<br>& AU 4462093 A & AU 625379 B<br>& AU 629742 B & AU 629743 B<br>& AU 630028 B & AU 632776 B<br>& AU 637777 B & AU 640355 B<br>& AU 642491 B & AU 652463 B<br>& AU 654874 B & AU 673014 B<br>& DE 69308941 D & DE 69211066 C<br>& DE 69313549 C & DE 69523518 D<br>& DE 69108133 C & DE 3802168 A<br>& DE 3942643 A & DE 3942642 A<br>& DE 3942641 A & DE 3942644 A<br>& DE 69211066 T & FI 896316 A<br>& FI 896317 A & FI 896318 A<br>& FI 880425 A & FI 913199 A<br>& FI 913200 A & FI 923466 A<br>& FI 946119 A & FR 2610195 A<br>& FR 2640134 A & FR 2641186 A<br>& FR 2641187 A & FR 2647010 A<br>& FR 2647011 A & FR 2647013 A<br>& FR 2651235 A & FR 2651124 A<br>& FR 2669532 A & FR 2681529 A<br>& FR 2684550 A & GB 2200551 A<br>& GB 2227660 A & GB 2227661 A<br>& GB 2230187 A & GB 2230188 A<br>& GB 2230189 A & GB 2235201 A<br>& GB 2235133 A & GB 2257362 A<br>& GB 2259856 A & GB 2263066 A<br>& CA 2006703 A & CA 2006706 A<br>& CA 2006713 A & CA 2006716 A<br>& CA 2006717 A & CA 2006719 A<br>& CA 2006707 A & CA 2006718 A<br>& CA 2046013 A & CA 2046012 A<br>& CA 2075095 A & CA 2099898 A<br>& CA 2104532 A & CA 1327942 A<br>& CA 1328081 A & CA 1328623 A<br>& CA 2153762 A & CA 2175966 A<br>& CA 1339301 A & BE 1001110 A<br>& BE 1004240 A & BE 1004366 A<br>& BE 1005904 A & BE 1006029 A<br>& BE 1007179 A & NL 8800206 A<br>& NL 8903186 A & NL 8903187 A<br>& NL 8903188 A & NL 8903185 A<br>& SE 9703714 D & SE 8800299 A<br>& SE 8904179 A & SE 8904180 A<br>& SE 8904181 A & SE 9703681 A<br>& SE 9703715 A & SE 510832 C | |

Form PCT/ISA/210 (continuation of second sheet) (July 1998)

**INTERNATIONAL SEARCH REPORT**

International application No.
PCT/JP02/07066

C (Continuation). DOCUMENTS CONSIDERED TO BE RELEVANT

| Category* | Citation of document, with indication, where appropriate, of the relevant passages | Relevant to claim No. |
|---|---|---|
| | & SE 5103702 C & IT 1219830 B<br>& IT 1237483 B & IT 1237484 B<br>& IT 1238355 B & IT 1241168 B<br>& RO 110299 A & RU 2066180 C<br>& RU 2085184 C & RU 2089176 C<br>& RU 2097021 C & RU 2097019 C<br>& RU 2103990 C & RU 2116781 C<br>& RU 2132182 C & AT 296789 A<br>& AT 296889 A & AT 18688 A<br>& AT 395109 B & AT 397765 B<br>& AT 398034 B & AT 296689 A<br>& AT 119764 T & AT 138557 T<br>& AT 150291 T & AT 157533 T<br>& AT 207731 T & HU 52695 A<br>& HU 9300151 D & HU 9300153 D<br>& HU 54486 A & HU 9300152 D<br>& HU 58507 A & HU 912220 A<br>& HU 52694 A & HU 206971 B<br>& HU 61892 A & HU 212964 B<br>& CZ 8907511 A & CZ 8907512 A<br>& CZ 8907509 A & CZ 283212 B<br>& CZ 8907510 A & DK 37188 A0<br>& DK 37188 A & DK 670989 A<br>& DK 671089 A & DK 671189 A<br>& DK 671289 A & DK 469722 T<br>& DK 525913 T & NO 301214 T<br>& NO 880410 A & NO 895309 A<br>& NO 895310 A & NO 895311 A<br>& NO 912589 A & NO 912590 A<br>& NO 932571 A & ES 1004562 U<br>& ES 2010740 A & ES 2023294 A<br>& ES 2023295 A & ES 2023296 A<br>& ES 2023297 A & ES 2072545 T<br>& ES 2090485 T & ES 2101939 T<br>& PT 86661 A & PT 92733 A<br>& PT 92734 A & PT 92735 A<br>& PT 92736 A & PT 98165 A<br>& PT 100739 A & PT 98164 A<br>& PT 101300 A & PT 101342 A<br>& PT 92734 B & PT 92733 B<br>& PT 92736 B & GR 88100046 A<br>& GR 89100851 A & GR 89100852 A<br>& GR 89100853 A & GR 89100854 A<br>& GR 91100287 A & GR 91100288 A<br>& GR 92100345 A & GR 93100308 A<br>& GR 93100349 A & NZ 231812 A<br>& NZ 223125 A & NZ 236641 A<br>& NZ 236642 A & NZ 236643 A<br>& NZ 236644 A & NZ 236645 A<br>& NZ 238146 A & NZ 231813 A<br>& NZ 231814 A & NZ 238699 A<br>& NZ 247798 A & NZ 243659 A<br>& NZ 248409 A & NZ 238700 A<br>& IL 92691 D & IL 92692 D<br>& IL 92693 D & IL 92694 D | |

Form PCT/ISA/210 (continuation of second sheet) (July 1998)

**INTERNATIONAL SEARCH REPORT**

International application No.
PCT/JP02/07066

C (Continuation). DOCUMENTS CONSIDERED TO BE RELEVANT

| Category* | Citation of document, with indication, where appropriate, of the relevant passages | Relevant to claim No. |
|---|---|---|
| | & IL 98606 D & IL 98607 D<br>& ZA 8800130 A & ZA 8909970 A<br>& ZA 8909971 A & ZA 8909972 A<br>& ZA 8909973 A & ZA 9104896 A<br>& ZA 9104989 A & ZA 9205661 A<br>& ZA 9304930 A & ZA 9305574 A<br>& ZA 9303908 A & ZA 9505520 A<br>& EG 19073 A & EG 19386 A<br>& EG 19074 A & EG 19193 A<br>& TR 26246 A & TR 26141 A<br>& TR 27500 A & TR 27614 A<br>& TR 28621 A & LU 87650 A<br>& LU 87651 A & LU 87652 A<br>& PL 290927 A & PL 290909 A<br>& PL 163551 B & PL 165411 B<br>& PL 165470 B & PL 169998 B<br>& DD 291244 A & DD 291245 A<br>& DD 291246 A & IE 63176 L<br>& IE 65326 L & IE 894196 A<br>& IE 912317 A & IE 912318 A<br>& IE 922541 A & IE 63176 B<br>& IE 65326 B & IE 65678 B<br>& CH 679639 A & CH 679674 A<br>& CH 679741 A & CH 680111 A<br>& CS 9102036 A & CS 9102037 A<br>& OA 9253 A & OA 9254 A<br>& OA 9256 A & ZW 17289 A<br>& SK 233192 A & SK 751289 A<br>& SK 750989 A & SK 751089 A<br>& MX 163856 B & MX 9100614 A<br>& MX 9204292 A & MX 172420 B<br>& MX 9304452 A & MX 173741 B<br>& MX 9304103 A & MX 9303474 A<br>& BR 8906850 A & BR 8906866 A<br>& BR 8906854 A & BR 9102759 A<br>& BR 9102766 A & BR 9202951 A<br>& BR 9302362 A & BR 9302881 A<br>& BR 9303433 A & BR 9602269 A<br>& IN 168400 A & IN 173866 A<br>& PH 24874 A & PH 26686 A<br>& SG 89194 G & SG 89294 G<br>& SG 89394 G & SG 89194 A<br>& SG 89294 A & SG 89394 A<br>& SG 51994 G & HK 102394 A<br>& HK 102494 A & HK 102594 A<br>& HK 89194 A & HK 172095 A<br>& HK 154796 A & HK 154896 A<br>& HK 154996 A & HK 70497 A<br>& HK 70597 A & HK 70697 A<br>& HK 1007493 A & KR 9108747 Y<br>& KR 9208199 Y & KR 155985 B<br>& KR 156549 B & KR 239315 B<br>& CN 1044047 A & CN 1044223 A<br>& CN 1049669 A & CN 1049606 A<br>& CN 1057778 A & CN 1057779 A | |

Form PCT/ISA/210 (continuation of second sheet) (July 1998)

**INTERNATIONAL SEARCH REPORT**

International application No.
PCT/JP02/07066

C (Continuation). DOCUMENTS CONSIDERED TO BE RELEVANT

| Category* | Citation of document, with indication, where appropriate, of the relevant passages | Relevant to claim No. |
|---|---|---|
| | & CN 1077113 A & ZM 5089 A<br>& ZM 5189 A & ZM 5289 A | |
| A | JP 7-165550 A (Lion Corp.),<br>27 June, 1995 (27.06.95),<br>Claims<br>(Family: none) | 8-13 |
| A | EP 953347 A2 (BASF AG),<br>03 November, 1999 (03.11.99),<br>Claims<br>& DE 19814739 A & DE 19814739 A1<br>& US 6271307 B1 & JP 11-343250 A | 8-13 |
| A | JP 11-12144 A (Lion Corp.),<br>19 January, 1999 (19.01.99),<br>Claims<br>(Family: none) | 8-13 |

Form PCT/ISA/210 (continuation of second sheet) (July 1998)